# EUROPEAN PATENT APPLICATION

(11) **EP 3 388 024 A1**
(43) Date of publication of application: **17.10.2018**
(21) Application number: 18156283.6
(22) Date of filing: 12.02.2018
(51) Int. Cl.: A61F 2/01

(54) **FILTER FOR CAPTURING THROMBI**

(30) Priority: 22.02.2017 US 201762461906 P
(71) Applicant: Cook Medical Technologies LLC, Bloomington, IN 47404 (US)
(72) Inventor: KOLD, Jens, 4623 Lille Skensved (DK); JOHNSEN, Jeppe Bockhaus, 5871 Froerup (DK); OSBAECK, Susan, 4191 Glumsoe (DK); KLAUSEN, Kasper, 4623 Lille Skensved (DK)
(74) Representative: Garratt, Peter Douglas

(57) **Abstract**

Disclosed herein is a removable vena cava filter for capturing thrombi in a blood vessel. The filter includes a plurality of primary struts having first ends attached together at their first ends. The filter also includes a plurality of secondary struts spaced between the primary struts and having third ends attached together along the longitudinal axis. Each secondary strut freely extends from the third end to a fourth end avoiding contact with other secondary struts and primary struts. The filter has an expanded configuration, in which the second arcs of the plurality of secondary struts may define a cavity having a hemiovoid shape, such that an ovoid formed from a reflection of the hemiovoid shape of the cavity does not extend beyond the end boundary of the filter defined by the second ends of the primary struts.

## Description

### BACKGROUND OF THE INVENTION

The present invention relates to medical devices. More particularly, the invention relates to a removable filter that can be percutaneously placed in and removed from a body vessel of a patient.

Filtering devices that are percutaneously placed in the vena cava have been available for a number of years. A need for filtering devices arises in trauma patients, orthopedic surgery patients, neurosurgery patients, or in patients having medical conditions requiring bed rest or non-movement. During such medical conditions, the need for filtering devices arises due to the likelihood of thrombosis in the peripheral vasculature of patients wherein thrombi break away from the vessel wall, risking downstream embolism or embolization. For example, depending on the size, such thrombi pose a serious risk of pulmonary embolism wherein blood clots migrate from the peripheral vasculature through the heart and into the lungs. A filtering device can be deployed in the vena cava of a patient when, for example, anticoagulant therapy is contraindicated or has failed.

Filtering devices are generally securely implanted in a vessel so as to minimize or avoid migration of the device as a result of blood pressure or vessel movement. Implantable devices are often provided with anchoring elements, usually in the form of barbs, attached to the extremities of at least some of the elements of the device, which anchor into the vessel wall. For this to be achieved reliably, it is necessary for the device to press against the vessel wall.

Normally, a vena cava filter is placed percutaneously through access in the jugular or the femoral vein using the Seldinger technique. After placing a pre-dilator and an introducer sheath via a guide wire and verifying the position of the filter with a cavogram, the filter is implanted. The filter is implanted by pushing an introducer system that carries the filter through the introducer sheath end, and when at the intended location, releases the filter.

Intravascular filters are designed to have a preferred alignment in which filtering is thought to be optimal. Although a misaligned filter may still be capable of filtering blood and catching clots or thromboemboli, it is preferable that the filter be implanted and centered in such a way that filtering capacity is maximized.

It has been a challenge to design a vena cava filter with features that facilitate centering within a blood vessel during and after deployment while maintaining the effectiveness of the filter.

### BRIEF SUMMARY OF THE INVENTION

One embodiment of the present invention generally provides a removable vena cava filter a plurality of primary struts having first ends attached together along a longitudinal axis. Each primary strut extends from the first end arcuately with respect to the longitudinal axis to a second end to define a first length. The arcuate segment including a first curved portion and a second curved portion. The first curved portion extends from the first end away from the longitudinal axis. The second curved portion extends from the first curved portion toward the longitudinal axis and terminating the second end. The plurality of second ends define a device end of the filter. A plurality of secondary struts are spaced between the primary struts and having third ends attached along the longitudinal axis. Each secondary strut extends from the third end to a fourth end to define a second length less than the first length. Each secondary strut extends arcuately with respect to the longitudinal axis and linearly radially. Each secondary strut includes a first arc and a second arc. The first arc extends from the third end away from the longitudinal axis. The second arc extends from the first arc toward the longitudinal axis and terminating at the fourth end. The filter is movable between a collapsed configuration and an expanded configuration. When the filter is in the expanded configuration, the second arcs of the plurality of secondary struts define a cavity having a hemiovoid or hemiellipsoid shape, such that an ovoid or ellipsoid formed from a reflection of the respective hemiovoid or hemiellipsoid shape of the cavity does not extend beyond the end boundary of the filter defined by the second ends of the primary struts.

In another embodiment, the present disclosure generally provides a removable vena cava filter a plurality of primary struts having first ends attached together along a longitudinal axis. Each primary strut extends from the first end arcuately with respect to the longitudinal axis to a second end to define a first length. The arcuate segment including a first curved portion and a second curved portion. The first curved portion extends from the first end away from the longitudinal axis. The second curved portion extends from the first curved portion toward the longitudinal axis and terminating the second end. The plurality of second ends define a device end of the filter. A plurality of secondary struts are spaced between the primary struts and having third ends attached along the longitudinal axis. Each secondary strut extends from the third end to a fourth end to define a second length less than the first length. Each secondary strut extends arcuately with respect to the longitudinal axis and linearly radially. Each secondary strut includes a first arc and a second arc. The first arc extends from the third end away from the longitudinal axis. The second arc extends from the first arc toward the longitudinal axis and terminating at the fourth end. The filter is movable between a collapsed configuration and an expanded configuration such that, when the filter is in the expanded configuration and constrained within the body vessel, the anchoring hooks of the primary struts and the second arcs of the secondary struts engage the wall of the body vessel, a second radial force exerted by each secondary strut is at least equal to a first radial force exerted by each primary.

In another embodiment, the present disclosure generally provides a removable vena cava filter a plurality of primary struts having first ends attached together along a longitudinal axis. Each primary strut extends from the first end arcuately with respect to the longitudinal axis to a second end to define a first length. The arcuate segment includes a first curved portion and a second curved portion. The first curved portion extends from the first end away from the longitudinal axis. The second curved portion extends from the first curved portion toward the longitudinal axis and terminating the second end. The plurality of second ends define a device end of the filter. A plurality of secondary struts are spaced between the primary struts and having third ends attached along the longitudinal axis. Each secondary strut extends from the third end to a fourth end to define a second length less than the first length. Each secondary strut extends arcuately with respect to the longitudinal axis and linearly radially. Each secondary strut includes a first arc and a second arc. The first arc extends from the third end away from the longitudinal axis. The second arc extends from the first arc toward the longitudinal axis and terminating at the fourth end. The filter is movable between a collapsed configuration and an expanded configuration. Each of the plurality of primary struts has a first thickness, and each of the plurality of secondary struts has a second thickness substantially equal to, or less than, the first thickness

Further aspects, features, and advantages of the invention will become apparent from consideration of the following description and the appended claims when taken in connection with the accompanying drawings.

### BRIEF DESCRIPTION OF THE DRAWINGS

FIG. 1 is a side view of a filter in an unconstrained, expanded configuration;
FIG. 2 is a comparative view of the filter of FIG. 1 compared to a prior art filter;
FIG. 3 is a view of a portion of a filter device and an interposed geometric solid;
FIG. 4 is a view of one strut of a vena cava filter and the geometric solid of FIG. 3;
FIG. 5 is a view of the ends of the secondary struts of a vena cava filter;
FIG. 6 is another view of a filter device and an interposed geometric solid;
FIG. 7 is a geometric view of a secondary strut of a filter in accordance with the principles of the present disclosure;
FIG. 8 is a graphical representation of radial force generated by the struts of a filter in accordance with the principles of the present disclosure;
FIGs. 9A-9D are illustrations of steps of the implantation or withdrawal of a filter in accordance with the principles of the present disclosure;
FIG. 10 is a view of the end of a primary strut in accordance with the principles of the present disclosure;
FIG. 11 is a view of the end of a primary strut in accordance with the principles of the present disclosure;
FIG. 12 is a view of a filter in accordance with another embodiment of the present disclosure;
FIG. 13 is a view of a portion of the filter of FIG. 12; and
FIG. 14 is an end view of a filter constructed in accordance with another embodiment of the present invention.

### DETAILED DESCRIPTION OF THE INVENTION

"About" or "substantially" mean that two given quantities (e.g. lengths, areas or volumes) are within 10%, preferably within 5%, more preferably within 1%. For example, a first quantity of length can be within 10% of a second length quantity.

"Adjacent" referred to herein is near, near to, or in close proximity with.

"Longitudinally" and derivatives thereof will be understood to mean along the longitudinal axis of the device or device assembly. When the device is implanted in a body vessel, "longitudinal" may instead pertain to an axis running centrally through the body vessel, which in the instance of an untilted filter will be at least partially coincident with "longitudinal" as pertains to the device axis.

"Curve," as used herein, means a bend in a rounded fashion, in contrast to an angle, which includes two straight segments meeting at or emanating from a vertex.

A structure having a "soft S-shape" includes two curves arranged substantially as the curves of the letter S, with each curve having a radius of curvature greater than the length of the structure.

The terms "proximal" and "distal" and derivatives thereof will be understood in the frame of reference of a device implanted within a body vessel. Thus, in some cases, particularly during retrieval of the filter, proximal refers to locations closer to the physician and distal refers to the locations farther away from the physician (e.g., deeper in the patient's vasculature).

The term "biocompatible" refers to a material that is substantially nontoxic in the in vivo environment of its intended use, and that is not substantially rejected by the patient's physiological system (i.e., is non-antigenic).

As used herein, the term "body vessel" means any body passage lumen that conducts fluid, including but not limited to blood vessels, esophageal, intestinal, biliary, urethral and ureteral passages.

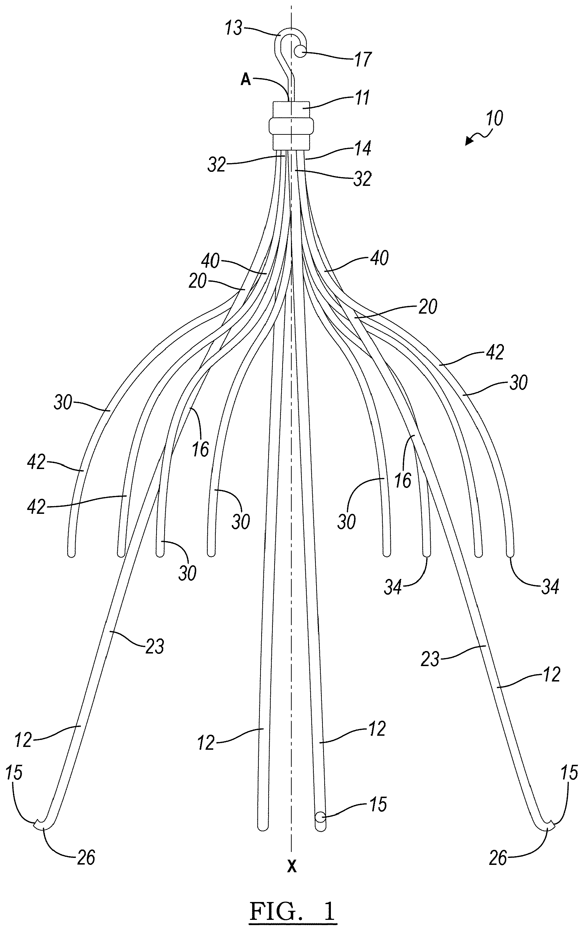

In accordance with one embodiment of the present invention, FIG. 1 illustrates a filter 10 in an unconstrained, expanded state. In this embodiment, the filter 10 includes four primary struts 12 each having first ends that are contained within a collet, or hub 11. In one embodiment, collet or hub 11 may be attached by crimping first ends 14 of primary struts 12 together at a center point A in a compact bundle along a central or longitudinal axis X of the filter. The hub 11 has a minimal diameter to securely contain the wire used to form the struts.

In one embodiment, the primary struts 12 may be formed of a superelastic material, stainless steel wire, Nitinol, colbalt-chromium-nickel-molybdenum-iron alloy, cobalt-chrome alloy, or any other suitable superelastic material that will result in a self-opening or self-expanding filter. In this embodiment, the primary struts 12 are preferably formed from wire having a round cross-section with a diameter of at least about 0.30 millimeters (mm) to about 0.60 mm, or about 0.36 mm to about 0.45 mm. It is not necessary that the primary struts have a round or near round cross-section.

Each primary strut 12 may include an arcuate segment 16 having a soft S-shape. Each arcuate segment 16 is formed with a first curved portion 20 that is configured to softly bend away from the longitudinal or central axis X of the filter 10 and a second curved portion 23 that is configured to softly bend toward the longitudinal axis of the filter 10. Due to the soft bends of each arcuate segment 16, a prominence or a point of inflection on the primary strut 12 is substantially avoided to aid in non-traumatically engaging the vessel wall. In some embodiments, a primary strut 12 may also include a third arcuate segment for formation of a barb or a hook element.

In another embodiment, the primary struts 12 may include an angle, such as an obtuse angle, or may be substantially straight segments.

In certain embodiments, the primary struts 12 may include anchoring hooks 26 that will anchor in the vessel wall when the filter 10 is deployed at a delivery location in the blood vessel. In one embodiment, and as illustrated in FIG. 1, the primary struts 12 terminate at anchoring hooks 26 at end 15. In other embodiments, the end 15 may be a substantially blunt end, and the anchoring hooks 26 may be formed at, or attached to, another point along the length of the primary struts 12. The primary struts 12 are configured to move as the filter 10 moves between an expanded state for engaging the anchoring hooks 26 with the blood vessel and a collapsed state for filter retrieval or delivery.

In some embodiments, such as when the primary struts 14 terminate with anchoring hooks 26, the strut ends 15 will not represent an end 90 of the device. In other embodiments, such as when the anchoring hooks 26 lie along a length of a primary strut 14, the strut ends 15 may represent an end 90 of the device.

In the expanded state, each arcuate segment 16 extends arcuately with respect to the longitudinal axis X (as shown in FIG. 1) and linearly relative to a radial direction as the path of the primary strut 12 is followed from the first end 14 to the anchoring hook 26.

In an embodiment in which the primary struts 12 have a soft S-shape, the soft bends of each arcuate segment 16 allow each primary strut 12 to cross another primary strut 12 along the longitudinal axis X in the collapsed state such that each anchoring hook 26 faces the longitudinal axis X for filter retrieval or delivery.

When the filter 10 is deployed in a blood vessel, the anchoring hooks 26 engage the walls of the blood vessel to define a first axial portion to secure the filter in the blood vessel. The anchoring hooks 26 prevent the filter 10 from migrating from the delivery location in the blood vessel where it has been implanted. The primary struts 12 are shaped and dimensioned such that, when the filter 10 is freely expanded in an unconstrained fashion, the filter 10 has a diameter of between about 25 mm and about 45 mm, or between about 30 mm and about 35 mm. The filter 10 may also have a length from the collet 11 to the furthest end of the primary struts 12 of between about 3 centimeters (cm) and about 7 cm, or about 4 cm to about 6 cm, or about 4.3 cm to about 4.5 cm. For example, the filter 10 may have a diameter of about 35 mm and a length of about 4.5 cm.

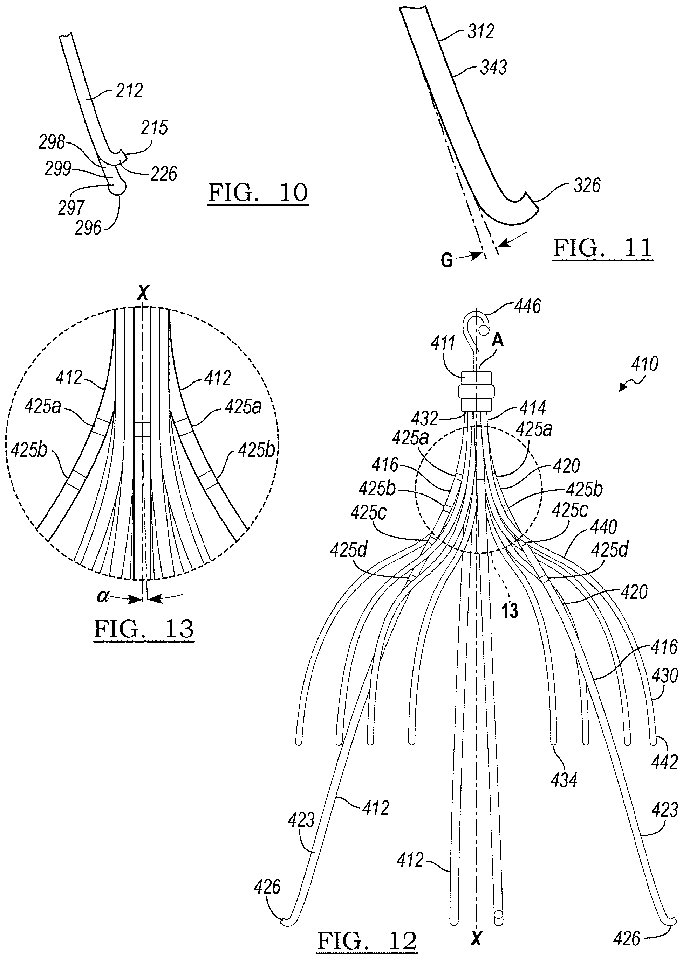

The primary struts may have a structure as illustrated in FIG. 10, where primary strut 214 is illustrated. In this embodiment, the strut 214 has an end 215 and a hook 226 at the end 215. The strut 214 is also provided with a stop member 299, which limits the extent to which the hook 226 can embed into the wall of a vessel in which it is implanted. A number of designs for stop members are disclosed in U.S. Patent Publication No. 2016/0067030, which is incorporated herein by reference in its entirety.

The filter 10 further includes a plurality of secondary struts 30 having connected ends 32 that independently emanate from hub 11. Hub 11 attaches by crimping the connected ends 32 at the center point A of the secondary struts 30 together with the primary struts 12. In this embodiment, each primary strut 12 has two secondary struts 30 in side-by-side relationship with the primary strut 12. The secondary struts 30 independently extend from the third ends 32 to fourth ends 34 to centralize the filter 10 in the expanded state in the blood vessel. The fourth ends 34 may be free ends unconnected to another portion of the device. The third ends 32 may be connected ends which occupy the interior of collet or hub 11; that is, the hub 11 may house a bundle of first ends 14 of the primary struts 14 and third ends 32 of secondary struts 30.

As shown in FIG. 1, each secondary strut 30 freely extends from the hub 11, avoiding contact with other struts. This configuration lessens the likelihood of entanglement. As shown, each secondary strut 30 extends arcuately along a longitudinal axis and linearly relative to a radial axis from the third end 32 to the fourth end 34 for engaging the anchoring hooks 26 with the blood vessel. As with the primary struts 12, the secondary struts 30 extend linearly relative to the radial axis and avoid entanglement with other struts.

The secondary struts 30 may be made from the same type of material as the primary struts 12. In this embodiment, each of the secondary struts 30 is formed of a first arc 40 and a second arc 42. The first arc 40 extends from the third end 32 away from the longitudinal axis X. The second arc 42 extends from the first arc 40 towards the longitudinal axis X. As shown, two secondary struts 30 are located on each side of one primary strut 12 to form the filtering portion of the filter 10. In one embodiment, the hub or collet 11 may be made of the same material as the primary struts and secondary struts to minimize the possibility of galvanic corrosion when implanted.

When freely expanded, fourth ends 34 of the secondary struts 30 will expand radially outwardly to a diameter of about 25 mm to about 45 mm, or about 30 mm to about 35 mm, to engage the vessel wall. The second arcs 42 of the fourth ends 34 engage the wall of a blood vessel to define a second axial portion, where the vessel wall is engaged by at least a portion of the second arcs 42. In certain embodiments, the extreme (fourth) end of a secondary strut 30 may curve inward, pointing back to the center of the vessel into which it has been implanted, but a more upstream portion of the second arc 42 may contact the vessel wall. The secondary struts 30 function to stabilize the position of the filter 10 about the center of the blood vessel in which it is deployed. As a result, the filter 10 has two layers or portions of struts longitudinally engaging the vessel wall of the blood vessel. In the illustrated embodiment, the length of the filter 10 is effectively defined by the length of a primary strut 12, as the secondary struts 30 do not extend beyond the ends 15 of the primary struts 12.

It is to be noted that any state in which the filter 10 takes on a generally conical or open shape may be considered an expanded state. In particular, an "unconstrained" expanded state is realized when the filter 10 is entirely unconstrained, when it is in neither the delivery system nor the vessel in which it is intended to be implanted, such as when sitting on a tabletop. However, when implanted, the filter 10 is also in an expanded state. It will be appreciated by one of skill in the art that the unconstrained expanded state permits the filter to have larger width/diameter dimensions than the inward force cause by pushing against a vessel wall will permit in the implanted expanded state.

As shown in FIG. 1, the diameter of the hub 11 may be defined by the size of a bundle containing the primary struts 12 and secondary struts 30. As shown, removal hook 13 extends from hub 11 opposite primary and secondary struts 12 and 30. The removal hook 13 may terminate in a blunt member such as sphere 17 in order to minimize or eliminate trauma from the end of the hook to the vasculature in which the device is implanted.

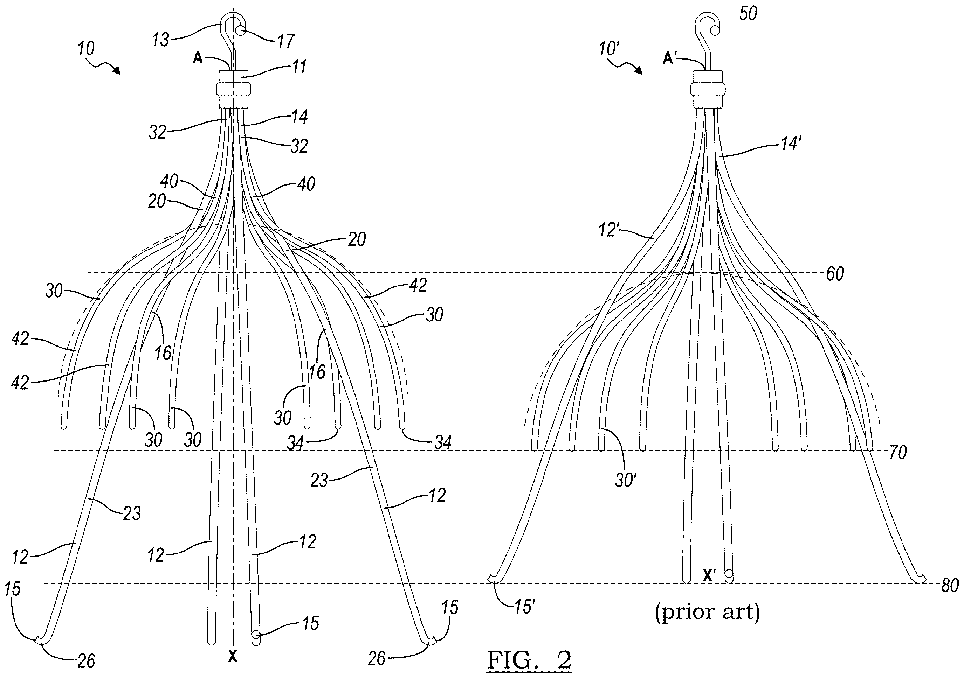

FIG. 2 illustrates a comparison between a prior art filter 10' and a filter 10 constructed according to the principles of the present disclosure. The furthest extreme points of the retrieval hooks 13/13' are aligned as shown by line 50. An arcing shape defining a curve extending from one second arc 42/42' of a pair of opposing secondary struts 30/30' is shown in a dashed line. Line 60 is tangent the arc of filter 10'. As can be seen in FIG. 2, the arc formed by the secondary struts 30 of filter 10 has its apex closer to hub 11 than does the arc formed by the secondary struts 30' of filter 10'.

Line 70 is drawn at the point where fourth ends 34' of filter device 10' align. In this embodiment, it can be seen from FIG. 2 that the fourth ends 34 of filter device 10 terminate proximal closer to the hub 11 than in device 10'. However, in other embodiments, it may be desirable to have longer extensions on the secondary struts 30 that will extend in a longitudinal direction away from the hub.

Line 80 is drawn at the second ends 15' of filter 10'. The primary struts 12 of filter 10 extend beyond the line 80 to a furthest point 90. In the depicted embodiment, second ends 15 define an overall longer filter device 10 in the case of the filter that accords with the principles of the present disclosure. In another embodiment, the length of the filter device 10 may be substantially equal to that of prior art device 10'.

Taken together, the features of the present invention give rise to a filter 10 in which both the primary struts 12 and the secondary struts 30 make a substantial contribution to the radial force exerted against the wall of a body vessel in which the device 10 is implanted, which provides sufficient radial force to avoid migration of the device after implantation. The smaller radius of curvature of the second arcs 42 of the secondary struts 30 translates to a shorter distance from the hub 11 at which the secondary struts 30 reach an outer periphery of the device, and therefore form a wall-contacting portion of these struts 30. The distance between the wall-contacting portion of the secondary struts 30 and that of primary struts 12 (including, in some embodiments, hook 26) is increased relative to the prior art filter 10'. In certain embodiments, the fourth ends 34 of the secondary struts 30 may lie at a point about halfway between hub 11 and the second ends 15 of the primary struts 12. In other embodiments, the fourth ends 34 of the secondary struts 30 may lie at a point closer to the hub 11 than about halfway between hub 11 and the second ends 15 of the primary struts 12.

In some embodiments, the secondary struts 30 may include a substantially linear portion toward the fourth end 34. In such instances, the linear portion may be substantially parallel to the longitudinal axis X in the expanded state, either when deployed to a body vessel or an in an unconstrained configuration. Such a linear portion may allow for generation of greater radial force against a vessel to which the device is deployed, or may increase a wall-contacting portion of the device, or both. In some embodiments, this linear portion may have a length of about 1 mm to about 6 mm, or about 2 mm to about 5 mm, or about 3 mm. In another embodiment, the second arc 42 of at least one of the secondary struts 30 may continue such that the secondary struts 30 curve inward toward the longitudinal axis X, and, by extension, toward the center of the body vessel to which the filter 10 is deployed.

In certain embodiments, the primary struts 12 and the secondary struts 30 may be constructed of wires or wire segments which have the same thickness. A secondary strut 30 of such a construction allows for greater radial force generation. In this embodiment or in other embodiments, greater radial force is generated by the shorter strut length and the smaller radius of curvature of the strut. In one embodiment, the primary struts 12 and the secondary struts 30 may have a thickness of about 0.25 mm. In another embodiment, the primary struts 12 and the secondary struts 30 may have a thickness of about 0.36 mm. In other embodiments, the primary struts 12 and the secondary struts 30 may be constructed of wires or wire segments which have different thicknesses, including embodiments in which the secondary struts 30 are less thick than the primary struts 12. In one embodiment, the secondary struts have a thickness of about 0.24 mm, and the primary struts have a thickness of about 0.45 mm. In another embodiment, the secondary struts have a thickness of about 0.24 mm, and the primary struts may have a thickness of about 0.35 mm. The primary struts may be about twice as thick as the secondary struts, or about 1.5 times as thick as the secondary struts, or any ratio of between about 1 to 2 times as thick as the secondary struts, inclusive.

The amount of anticipated radial force to be generated by various struts may be adjusted by radial force balancing techniques. For example, filters of similar design may be constructed in subtly different ways in order to ensure that the radial force contributions of the primary struts and the secondary struts relative to one another are balanced as desired. In one example, a filter may realize an increase in radial force contribution from a secondary strut by incorporating secondary struts of the same length as a previous design, but using a thicker wire. Additionally, increasing the number of struts will increase the amount of radial force generated and allow for more contact between wire and the vessel wall after implantation of the device.

As mentioned, the primary struts 12, the secondary struts 30, and the collet 11 may be made of the same material. A shape memory metal may be particularly useful in the construction of the device 10. In particular, a cobalt-chrome alloy may be preferred. The device 10 may incorporate at least one radiopaque marker in order that implantation may be fluoroscopically tracked as the filter 10 is deployed, or alignment may be monitored after implantation. The radiopaque markers may be placed on the collet 11, or at the ends 15 of the primary struts 12, or along the secondary struts 30, or at an end of the retrieval hook 17, or any other portion of the device.

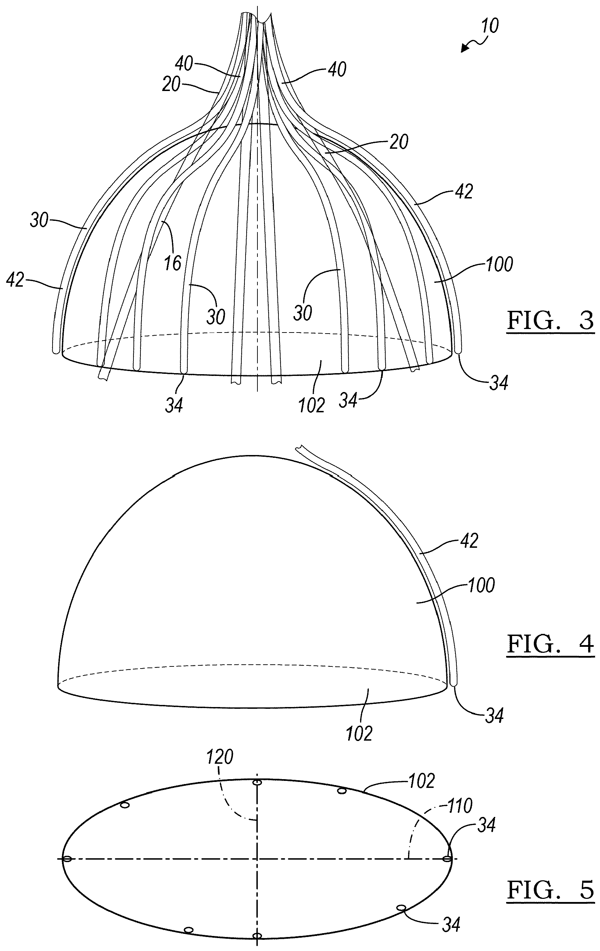

FIGs. 3-5 provide a detailed view of geometric properties of the secondary struts of a filter device 10 constructed in accordance with the principles of the present invention. A hemiovoid 100 with base 102 is depicted within the cavity of the filter. The hemiovoid 100 is defined by the radius of curvature of one of the second arcs 42 of a secondary strut 30. In the case where the device is perfectly symmetrical, all second arcs 42 will be coincident with the curvature of the ovoid 100. The base 102 is defined by the fourth ends 34 of the secondary struts 30 to form a generally round section. In certain embodiments, the hemiovoid may be a hemiellipsoid.

It should be noted that for the purposes of this disclosure that, a filter manufactured to be circular at the fourth ends 34 of the secondary struts 30, and therefore would encompass a hemisphere defined by the second arcs 42 of the secondary struts 30, would fall within the scope of the present disclosure, as the circle would be considered in this case to be a subset of the category of an ovoid in which the measure of all distances from the center of the solid to an edge would be equal (rather than being in a greater/lesser relationship.)

FIG. 4 demonstrates how a single second arc 42 defines the curvature of an oval, and thus a hemiovoid. The first arc 40 deviates away from the hemiovoid shape as it curves upward. Additionally, the hemiovoid 100 would conform to the same dimensions in an embodiment in which the secondary strut 30 illustrated has a linear extension toward distal end 34, as the hemiovoid only tracks the second arc 42. Additionally, in a filter 10 in which a first secondary strut 30 is paired with a second secondary strut 30 directly across from, or 180 degrees around from, first secondary strut, that the paired second arcs 42 of the paired secondary struts 30 may define the curvature of the same hemioval. This hemioval, when rotated about the longitudinal axis X of the device 10, would generate the hemiovoid 100 as illustrated. In the case of a device in which all secondary struts 30 are substantially identical, all second arcs 42 of all secondary struts 30 would define the curvature of such a solid 100.

FIG. 5 is a view of base 102 of hemiovoid 100 with the positions of secondary strut ends 34 indicated. In the illustrated embodiment, the base 102 represents a generally circular section of the full ovoid shape. The spacing of the secondary struts 30 is indicated only for one exemplary embodiment of the device 10; other arrangements are possible, including embodiments in which the secondary struts 30 are disposed at substantially equal distances from adjacent secondary struts.

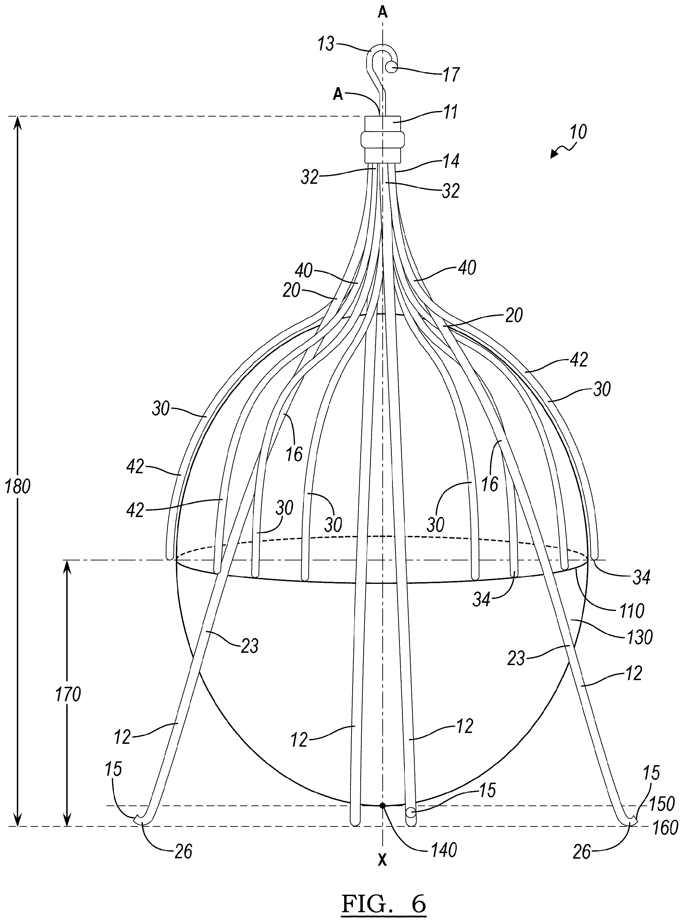

FIG. 6 illustrates a full view of device 10 with hemiovoid 100 interposed as in preceding figures. The hemiovoid 100 is generated by rotation of a curve as defined by the second arc 42 of a secondary strut 30 rotated around the longitudinal axis X in order to yield a hemiovoid 100 with base 110, which is substantially round in shape. To demonstrate the relative dimensions of the device, a reflection 130 across round base 110 of hemiovoid 100 is illustrated. The reflected hemiovoid 130 terminates at a furthest end 140. Tangent line 150 is drawn through the plane in which furthest end 140 lies. Line 160 is drawn through the furthest extremes of primary struts 12. It can be seen that line 150 lies closer to hub 11 than does line 160, meaning that the ovoid including both hemiovoid 100 and reflection 130 thereof lies within the cavity of device 10 formed of primary struts 12 and secondary struts 30 in the expanded state.

FIG. 6 indicates a distance 170 between the fourth ends 34 of secondary struts 30 and the furthest end of the device 10 as indicated by line 160. In some embodiments, this distance 170 may be about 10 mm to about 25 mm, or about 11 mm to about 20 mm, or about 12 mm to about 18 mm, or from about 13 mm to about 17 mm, or any value encompassed by any range. Distance 180 between the furthest edge 91 of collet 11 and the end 90 of the device 10 as defined by line 160. In some embodiments, this distance 180 may be about 35 mm to about 55 mm, or about 40 mm to about 50 mm, or about 43 mm to about 48 mm, or any value encompassed by any range. The overall length of the device 10 will be impacted by how much the filter is constrained by the bodily context into which it is implanted. As a result of the above dimensions, the distance between the fourth ends 34 of the secondary struts 30 and the second ends of the primary struts 14, when the device in an expanded, unconstrained state, may represent from about 25% to about 50% of the length of the device, or about 27% to about 40% of the length of the device, or about 30% to about 36% of the length of the device, or any value between about 25% and about 50% of the device length.

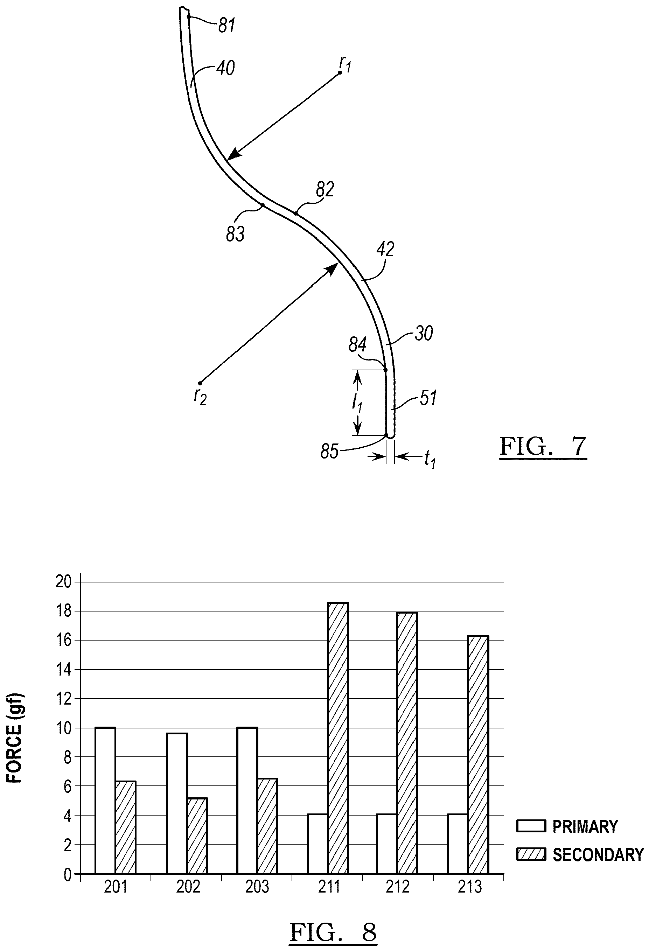

FIG. 7 further illustrates the geometric properties of a secondary strut 30 according to one embodiment of the present invention. In this embodiment, first arc 40 is defined between first point 81 and second point 82. The radius of curvature r1 of first arc 40 may be about 10 mm to about 12 mm. Second arc 42 is defined between third point 83 and fourth point 84. The radius of curvature r2 of second arc 42 may be about 10 mm to about 12 mm. The secondary strut 30 has linear portion 51 toward its fourth end 34, which lies further from hub 11 than the second arc 42. Linear portion 51 extends from fourth point 84 to fifth point 85 and defines length 11, which may measure about 1 mm to about 5 mm, or from about 2 mm to about 4 mm, or about 3 mm.

FIG. 8 demonstrates the radial force generation consequences of a filter design according to the present disclosure as compared with a prior art filter. Measurements are taken in triplicate, with filters 201/202/203 being of a prior design having thinner secondary struts than primary struts, and filters 211/212/213 being constructed according to the principles of the present invention, in which a diameter of a primary strut is less than that of the prior art device. The measurements illustrated were generated by 15 mm flat plate compression of primary and secondary struts. The measurements of two primary struts and four secondary struts are shown.

In the filters 201/202/203, two primary struts produce more radial force than four secondary struts. Each primary strut produces about 5 gram-force (gf) and each secondary strut about 1.5 gf, for a ratio of about 10:3 primary:secondary. In contrast, four secondary struts of filters 211/212/213 produce more radial force than two primary struts. In filters 211/212/213, each primary strut produces about 2 gf radial force, whereas each secondary strut produces about 4.5 gf, for a ratio of about 4:9. It may be desirable for a filter 10 of the present disclosure to be of such a construction that the amount of radial force produced by each secondary strut is substantially equal to that produced by each primary strut. In another embodiment, it may be preferable that the total radial force produced by all primary struts is substantially equal to that produced by all secondary struts. In another embodiment, the ratio of radial force produced by a primary strut 12 may be no more than twice that produced by a secondary strut. In another embodiment, each secondary strut 30 may produce at least twice the radial force of each primary strut 12.

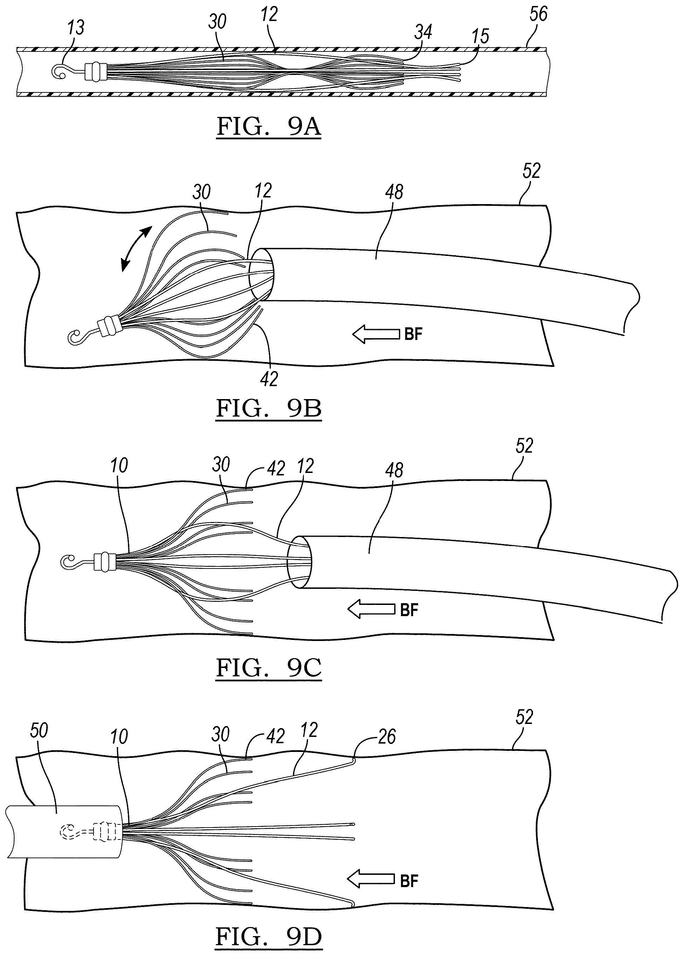

FIGs. 9A-9D illustrate a filter device 10 being delivered to and retrieved from a body vessel 52. The illustrated methods are exemplary; any suitable method may be used to deliver or retrieve a device 10 according to the principles of the present disclosure.

FIG. 9A illustrates the filter 10 in a collapsed configuration disposed in a delivery catheter 56 for delivery. As shown, the filter 10 is shaped for each primary strut 12 to cross another primary strut 12 along the longitudinal axis X. As a result, in the collapsed configuration, the anchoring hooks 26 invert or inwardly face the longitudinal axis X for delivery of the filter 10. This inverted or inwardly facing configuration of the anchoring hooks 26 allows for simplified delivery (and retrieval) of filter 10.

In the collapsed state, each primary strut 12 is configured to cross another primary strut 12 along the longitudinal axis X such that the arcuate segments 16, first curved portions 20 or second curved portions 23, occupy a first diameter D₁. In this embodiment, the first diameter is greater than a second diameter D₂ occupied by the anchoring hooks 26 for filter retrieval or delivery. It has been found that the first diameter of the arcuate segments 16 serves to clear a path of retrieval, reducing radial force from the sheath or blood vessel on the anchoring hooks 26 during removal of the filter 10 from a patient. The filter 10 may be delivered or retrieved by any suitable introducer system. In some embodiments, the introducer catheter may have an inside diameter of between about 4.5 French and 16 French, and more preferably between about 6.5 French and 14 French.

FIGs. 9B and 9C illustrate the filter 10 partially deployed in body vessel 52, which in some embodiments may be an inferior vena cava. FIG. 9B shows the filter 10 being delivered by catheter 48. For deployment of the filter 10, a delivery catheter is percutaneously inserted through the patient's vessel such that the distal end of the delivery catheter is at the location of implantation. In this embodiment, a wire guide may be used to guide the delivery tube to the location of deployment. In such an embodiment, a pusher wire having a pusher member at its distal end may be fed through an end of the delivery catheter 48 to a desired location within the lumen of the body vessel. The direction of blood flow in the vessel is indicated by the arrow labeled "BF."

FIG. 9B shows the filter 10 in an early stage of deployment. During deployment, the secondary struts 30 expand to centralize or balance the filter within the vessel 52. When the fourth ends of the secondary struts emerge from the end of either of the delivery catheter 48, the secondary struts 30 expand to an expanded configuration. The curvature of the secondary struts 30, namely in the portion of the second arcs 42, expand and with a "rolling" motion 92, engage the inner wall of the vessel 52 until the secondary struts 30 are as fully expanded as possible within the confines of the body vessel. This "rolling," facilitated by the relatively small radius of curvature of the second arcs 42, increases the probability that the device will be centered within the lumen of the body vessel.

As shown in FIG. 9C, as the delivery catheter 48 is further withdrawn to continue deployment of the filter, the device 10 achieves a largely centered position, and the primary struts 12 can be moved out of the catheter 48. FIG. 9D illustrates a deployed filter with the anchoring hooks 26 of the filter device 10 anchored into the inner wall of vessel 52, and in this embodiment, the second arcs 42 of the secondary struts 30 are also in engagement with the vessel wall. The anchoring hooks 26 of the primary struts 12 serve to anchor the filter 10 at the location of deployment in the vessel, preventing the filter 10 from moving with the blood flow through the vessel. As a result, the filter 10 is supported by two sets of struts that are spaced axially along the length of the filter. In some embodiments, the secondary struts 30 provide the majority of the radial force to maintain this positioning.

FIG. 9D illustrates the filter 10 in an expanded configuration but constrained within the body vessel 52. When the filter 10 is implanted, it may have an effective diameter of about 30 mm. Retrieval assembly 50 has been introduced to the vessel to remove the device 10. The hub 11 and removal hook 13 are positioned downstream from the location at which the anchoring hooks 26 are anchored in the vessel, relative to the direction of blood flow BF. When captured by the struts 12 and 30, thrombi remain lodged in the filter, and the filter 10 along with the thrombi may then be percutaneously removed from the vessel. When the filter 10 is to be removed, the removal hook 46 is preferably grasped by a retrieval instrument that is percutaneously introduced in the vena cava in the direction of removal hook 16 first. However, in other embodiments, a properly-sized retrieval catheter may be used such that the retrieval catheter 50 is advanced over the primary struts 12 and secondary struts 30, causing the device 10 to attain a collapsed configuration that allows for retrieval of the filter 10.

FIG. 10 illustrates a structural modification to a primary strut 212 which may be employed in conjunction with any other embodiment of the device as disclosed herein. In this instance, the primary strut 212 has a stop member 299 which includes a linear extension 297 and terminates in rounded end 296. The stop member 299 may be formed separately from the remainder of the primary strut 212 and may be attached such as by welding to the attachment point 298 at the position where the primary strut 212 has been bent to form the hook member 215. In some embodiments, the stop member 299 may be made of a radiopaque material, such as platinum or palladium, and may assist in visualization of the filter device during and after implantation. In some cases, the stop member 299 may function to modulate the degree of penetration of the vessel wall by the device. In a device in which stop members 299 are included, the ends of the stop member 299 may represent a device end of the device. Stop members are also disclosed in United States Patent No. 9,308,066, the entire contents of which are incorporated by reference.

FIG. 11 illustrates a structural modification to a primary strut 312 which may be employed in conjunction with any other embodiment of the device as disclosed herein. In this embodiment, the primary strut 312 is formed with a distal bend 343 away from the longitudinal axis of the device in a distal portion of the strut 312. The bend 343 away from the strut path G defined by a more hub-proximate portion of the strut 312 may be from about 0.5 degrees to about 10 degrees, or about 1 degree to about 7.5 degrees, or about 2 degrees to about 7 degrees, or about 6 to about 6.5 degrees.

FIGs. 12-14 illustrate a structural modification to a filter device 410 which may be employed in conjunction with any other embodiment of the device as disclosed herein. In this embodiment, each primary strut 412 may contain an axial bend 425. The position of the axial bend is generally within a first curved portion of a primary strut, as shown in potential bend positions 425a, 425b, 425c, and 425d. Typically, each primary strut 412 will have an axial bend at the same respective position as each other primary strut 412 of the device. The axial bends 425 of the respective primary struts 412 result in each primary strut 412 being angled by an angle α from a plane defined by the longitudinal axis X and a point along the section 416 of that particular primary strut 412. For example, referring to FIGs. 12-13, the axial bend 425 of the primary strut 412 is angled from the plane P by an angle α. The plane P is defined by the longitudinal axis X and a point along the section 416 that extends between the first end 414 of the primary strut 412 and the axial bend 425.

The angle of each axial bend 425 may range between about 0.5° and about 5°. As the primary struts 412 move between the closed state and the expanded state of the filter 410, the second curved distal portions 423 move radially, in a rotating fashion, from the longitudinal axis X toward the vessel wall. The rotating radial movement of the second curved distal portions 423 aid in reducing the risk of entanglement of the primary struts 412.

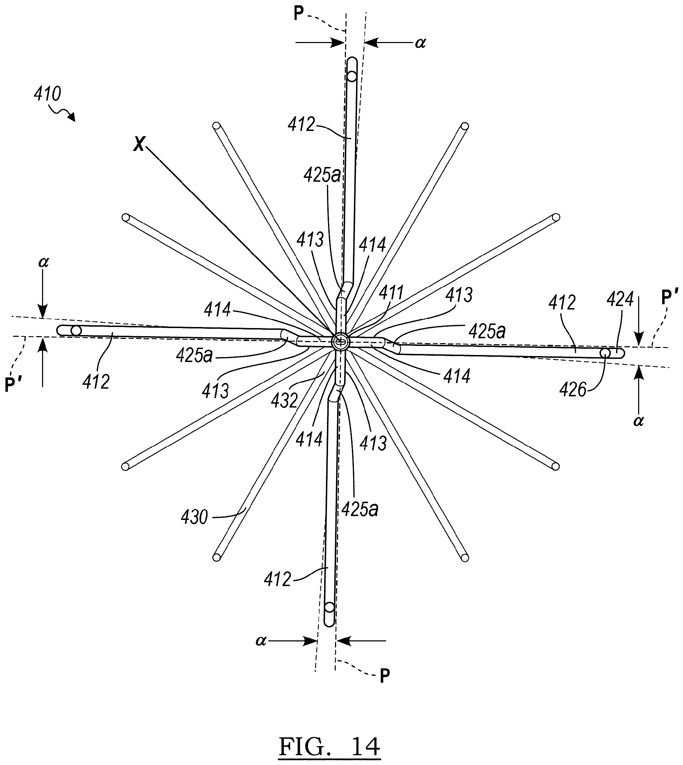

As shown in FIG. 14, the primary struts 412 extend substantially linearly along a diametric plane. However, the axial bend 425a on the first curved proximal portion 420 of each primary strut 412 results in each primary strut extending linearly along a diametric plane from the first end 414 to just before the axial bend 425a and from just after the axial bend 425a to the second end 426. Thus, the term "substantially linearly," as opposed to merely "linearly," takes into account this slight axial bend 425 on each of the respective primary struts 412. While the primary struts 412 do not extend completely linearly along a diametric plane due to the slight axial bends 425, they do not extend helically. It is noted that the axial bends 425 illustrated in FIG. 14 have been exaggerated for illustration purposes, but may not be so obvious when viewed with the naked eye. Further details about axial bends are disclosed in United States Patent No. 9,308,066, the entire contents of which are incorporated by reference.

Although the embodiments of this device have been disclosed as being constructed from wire having a round cross section, the struts could also take on other cross-sectional profiles, so long as the proper radial force and anchoring characteristics are maintained.

While the present invention has been described in terms of preferred embodiments, it will be understood, of course, that the invention is not limited thereto since modifications may be made to those skilled in the art, particularly in light of the foregoing teachings.

## Claims

1. A filter for capturing thrombi in a blood vessel, the filter comprising:
a plurality of primary struts having first ends attached together along a longitudinal axis, each primary strut extending from the first end arcuately with respect to the longitudinal axis to a second end to define a first length, the arcuate segment including a first curved portion and a second curved portion, the first curved portion extending from the first end away from the longitudinal axis, the second curved portion extending from the first curved portion toward the longitudinal axis to the second end, each of the plurality of primary struts having a first thickness; and
a plurality of secondary struts spaced between the primary struts and having third ends attached along the longitudinal axis, each secondary strut extending from the third end to a fourth end to define a second length less than the first length, each secondary strut extending arcuately with respect to the longitudinal axis and linearly radially, each secondary strut including a first arc and a second arc, the first arc extending from the third end away from the longitudinal axis, the second arc extending from the first arc toward the longitudinal axis and terminating at the fourth end, each of the plurality of secondary struts having a second thickness, the first thickness being about one to about two times the second thickness.

2. The filter of claim 1, wherein each second end is a free end terminating in an anchoring hook for engaging a wall of the blood vessel.

3. The filter of claim 1 or claim 2, wherein each fourth end is a free end not terminating in a hook.

4. The filter of any one of the preceding claims, further comprising a collet surrounding the first ends and the third ends to form a hub.

5. The filter of any one of the preceding claims, wherein the filter is movable between a collapsed configuration and an expanded configuration.

6. A filter for capturing thrombi in a blood vessel, the filter comprising:
a plurality of primary struts having first ends attached together along a longitudinal axis, each primary strut extending from the first end arcuately with respect to the longitudinal axis to a second end to define a first length, the arcuate segment including a first curved portion and a second curved portion, the first curved portion extending from the first end away from the longitudinal axis, the second curved portion extending from the first curved portion toward the longitudinal axis to an anchoring hook at the second end; and
a plurality of secondary struts spaced between the primary struts and having third ends attached along the longitudinal axis, each secondary strut extending from the third end to a fourth end to define a second length less than the first length, each secondary strut extending arcuately with respect to the longitudinal axis and linearly radially, each secondary strut including a first arc and a second arc, the first arc extending from the third end away from the longitudinal axis, the second arc extending from the first arc toward the longitudinal axis and terminating at the fourth end to define an extended wall-contacting portion;
the filter being movable between a collapsed configuration and an expanded configuration such that, when the filter is in the expanded configuration and constrained within the body vessel, the anchoring hooks of the primary struts and the second arcs of the secondary struts engage the wall of the body vessel, a second radial force exerted by each secondary strut is at least equal to a first radial force exerted by each primary strut.

7. The filter of claim 6, wherein the ratio of radial force exerted by one of the plurality of secondary struts is at least twice the radial force exerted by one of the plurality of primary struts.

8. A filter for capturing thrombi in a blood vessel, the filter comprising:
a plurality of primary struts having first ends attached together along a longitudinal axis, each primary strut extending from the first end arcuately with respect to the longitudinal axis to a second end to define a first length, each arcuate segment including a first curved portion and a second curved portion, the first curved portion extending from the first end away from the longitudinal axis, the second curved portion extending from the first curved portion toward the longitudinal axis to a second end, the plurality of second ends defining a device end of the filter; and
a plurality of secondary struts spaced between the primary struts and having third ends attached along the longitudinal axis, each secondary strut extending from the third end to a fourth end to define a second length less than the first length, each secondary strut extending arcuately with respect to the longitudinal axis and linearly radially, each secondary strut including a first arc and a second arc, the first arc extending from the third end away from the longitudinal axis, the second arc extending from the first arc toward the longitudinal axis and terminating at the fourth end;
the filter being movable between a collapsed configuration and an expanded configuration, wherein when the filter is in the expanded configuration, the second arcs of the plurality of secondary struts define a cavity having a hemiovoid shape, such that an ovoid formed from a reflection of the hemiovoid shape of the cavity does not extend beyond the end boundary of the filter defined by the second ends of the primary struts.

9. The filter of claim 8, wherein the fourth ends define the boundary of the largest cross-section of the hemiovoid shape.

10. The filter of any one of the preceding claims, wherein each second end is a free end terminating in an anchoring hook for engaging a wall of the blood vessel.

11. The filter of any one of the preceding claims, wherein each fourth end is a free end not terminating in a hook or anchoring structure.

12. The filter of claim 8 or claim 9, further comprising a collet surrounding the first ends and the third ends to form a hub.

13. The filter of any one of the preceding claims, wherein the hub further comprises a retrieval hook extending opposite the plurality of primary and secondary struts for removal of the filter from the blood vessel.

14. The filter of any one of the preceding claims, wherein when the filter is in the expanded configuration, the fourth ends of the secondary struts are positioned nearer the longitudinal axis than at least another portion of the second arc.

15. The filter of any one of the preceding claims, wherein each primary strut and secondary strut is formed of at least one of a superelastic material, a stainless steel wire, Nitinol, a cobalt-chromium-nickel-molybdenum-iron alloy, and a cobalt-chrome alloy.
